(19) Europäisches Patentamt
European Patent Office
Office européen des brevets

(11) EP 2 566 448 B1

(12) **EUROPEAN PATENT SPECIFICATION**

(45) Date of publication and mention of the grant of the patent:
**17.06.2015 Bulletin 2015/25**

(21) Application number: **11721664.8**

(22) Date of filing: **02.05.2011**

(51) Int Cl.:
***A61K 9/46*** *(2006.01)*
***A61K 47/00*** *(2006.01)*
***A61K 31/545*** *(2006.01)*

(86) International application number:
**PCT/TR2011/000127**

(87) International publication number:
**WO 2011/139252 (10.11.2011 Gazette 2011/45)**

(54) **EFERVESCENT FORMULATIONS COMPRISING CEFDINIR**

BRAUSEFORMULIERUNGEN MIT CEFDINIR

FORMULATIONS EFFERVESCENTES COMPRENANT DU CEFDINIR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.05.2010 TR 201003547**

(43) Date of publication of application:
**13.03.2013 Bulletin 2013/11**

(73) Proprietor: **Bilgic, Mahmut**
**34220 Esenler-Istanbul (TR)**

(72) Inventor: **Bilgic, Mahmut**
**34220 Esenler-Istanbul (TR)**

(56) References cited:
**WO-A1-2004/104010**
**DE-A1-102007 002 924**
**US-A1- 2005 131 079**
**US-A1- 2007 128 268**
**US-A1- 2007 134 325**

- **DATABASE WPI Week 200630 Thomson Scientific, London, GB; AN 2006-285271 XP002620814, & CN 1 706 389 A (JINAN PINGZHI MEDICINE SCI TECH CO LTD) 14 December 2005 (2005-12-14)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 566 448 B1

## Description

The present invention relates to effervescent pharmaceutical dosage forms comprising cefdinir as the active agent, and their preparation.

## Background of the Invention

The chemical structure of the molecule cefdinir, which has the chemical name (6R,7R)-7-[[(2Z)-(2-amino-4-thiazolil)(hydroxyimino)acetyl]amino]-3-ethenyl-8-oxo-5-thia-1-azabicyclo[4.2.0]oct-2-en-2-carboxylic acid and was first disclosed in the patent numbered BE897864, is shown in Formula I. This molecule, which is a third generation cephalosporin, is indicated for the treatment of many diseases caused by gram positive and gram negative bacteria.

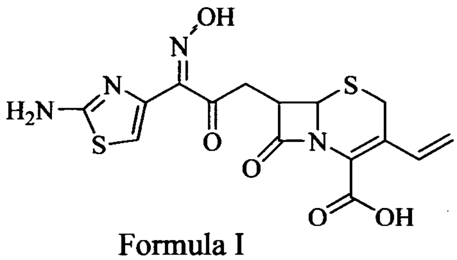

Formula I

Cefdinir which physically appears as a white powder has very poor solubility in common organic solvents such as methanol, ethanol, and acetonitrile and in water.

Cefdinir does not get wet or dissolve in water owing to its hydrophobic character. Moreover, cefdinir has a very bitter taste that it is difficult to mask it with flavoring agents or taste regulating agents.

The product under the name OMNICEF® is available in capsule and suspension forms on the market. Administration of solid oral dosage forms such as capsules, pills and tablets by oral route generally causes difficulty in swallowing for geriatric and pediatric patients. Although alternatively developed suspension dosage forms indicate higher bioavailability, the use of this dosage form brings along the possibility of excessive and/or uncontrolled dose intake particularly in pediatric and geriatric patients.

In the solid oral and suspension dosage forms, some methods have been tried in order to eliminate the bitter taste of cefdinir. In the tablets containing coating, it has been tried to mask the bitter taste of cefdinir with the help of the coating material. However, cefdinir has such a bitter taste that coating materials are unable to mask it. On the other hand, the coatings in the suspension dosage forms provide a solution for low solubility problem of cefdinir while they remain incapable of masking the bitter taste of it.

Alternatively, water dispersible forms are preferred since they are more convenient to use for geriatric and pediatric patients and they solve the solubility problem of cefdinir. Although some excipients such as taste regulating agent, aroma and/or flavoring agents are used to eliminate the bitter taste of cefdinir, a desirable and pleasant taste of it can not be achieved.

Accordingly, it is clearly seen that the problems such as difficulty in swallowing or low solubility of cefdinir can be eliminated to some extent. However, masking the bitter taste of cefdinir is a challenging task to overcome.

As it can be seen, new dosage forms and formulations need to be developed so as to provide reliable and user friendly dosage form alternatives for pediatric and geriatric patients, to overcome the solubility problems of cefdinir and especially, to eliminate the bitter taste of cefdinir.

The inventors have surprisingly found that the problems in the prior art can be solved by the pharmaceutical formulations formulated in effervescent form prepared according to the present invention.

## Description of the Invention

The subject of the present invention is effervescent formulations comprising cefdinir and the procedures related to their preparation. Surprisingly, it has been found that the effervescent formulations comprising cefdinir, wherein the combination of sodium chloride and sucralose is used as taste regulating agent and the ratio of sodium chloride to sucralose is in the range of 5:1 and 1:1 , have achieved to increase the solubility of cefdinir and eliminate the bitter taste of cefdinir.

The inventors have found that these effervescent formulations developed pertaining to the present invention can be administered by pediatric and geriatric patients easily, can lead to the increase in the solubility of cefdinir and can mask the bitter and durable taste of cefdinir entirely.

The choice of the taste regulating agent with respect to their water solubility values enables fine tuning of the solubility problem of cefdinir and the ratio of these two agents provides effective taste masking of the formed solution.

The term "effervescent formulations" present in the text comprises effervescent tablets, effervescent granules and effervescent powders.

Cefdinir that can be used in the effervescent formulations, which are the subject of the present invention, can be in the form of its solvates, hydrates, enantiomers, racemates, organic salts, inorganic salts, polymorphs, crystal and amorphous forms or in free form and/or combination thereof.

The inventors have found that the ratio of active agent to the taste regulating agent comprising two different agents is significant in masking the bitter taste of cefdinir. Therefore, it has been observed that unpleasant taste of cefdinir is successfully masked in the formulations in which the ratio of cefdinir to the combination of taste regulating agents is in the range of 10:1 to 1:1, preferably 8:1 to 1:1 and more preferably 5:1 to 2:1.

According to this, another aspect of the present invention is effervescent formulations wherein the ratio of cefdinir to the combination of taste regulating agents is in the range of 10:1 to 1:1, preferably 8:1 to 1:1 and more preferably 5:1 to 2:1.

In the present invention, the inventors have also found that the effervescent formulations comprising cefdinir, in which organic base is used in addition to cefdinir and the ratio of cefdinir to the organic base is in the range of 5:1 to 1:5, provide dissolution of cefdinir in water entirely. Therefore, the developed effervescent formulations pertaining to the present invention have both provided a high solubility of cefdinir in water and eliminated the unpleasant taste of cefdinir to a large extent.

According to this, another aspect of the present invention is the effervescent formulations comprising cefdinir in which organic base is used in addition to cefdinir and the ratio of cefdinir to the organic base is in the range of 5:1 to 1:5.

In the effervescent formulations pertaining to the present invention, primary amines, secondary amines, tertiary amines and/or heterocyclic compounds containing nitrogen can be used as the organic base in the present invention.

The organic base that is to be used in the formulation can be selected from, but not limited to, a group comprising ethanolamine, isopropanolamine, 1-dioxy-1-methylamino-sorbitol, 1-dioxy-1-methylamino-D-glucitol, tris(hydroxymethyl)aminomethane, N-(Tri(hydroxymethyl) methyl)glycine, N,N-Bis(2-hydroxyethyl)glycine, 2-methyl aminophenol. Preferably, 1-dioxy-1-methylamino-sorbitol and tris(hydroxymethyl)aminomethane are used.

Another aspect of the present invention is the effervescent formulations comprising pharmaceutically acceptable excipients in addition to the active agent cefdinir.

Various excipients selected from, but not limited to, a group comprising binders, lubricants, humectants, disintegrants, diluents, effervescent acid and effervescent base can be used in the effervescent formulation pertaining to the present invention apart from cefdinir, taste regulating agent and the organic base.

The binder that can be used in the effervescent formulations pertaining to the present invention can be selected from, but not limited to, a group comprising ethyl cellulose, gelatine, hydroxyethyl cellulose, hydroxymethyl cellulose, hydroxypropyl cellulose, hypromellose, magnesium aluminum silicate, methyl cellulose, povidone. Preferably, povidon is used in scope of the present invention.

The inventors have found that the dissolution time of the pharmaceutical formulation when 1-8% binder is used is shorter compared to the formulations comprising a higher amount of binder.

According to this, another aspect of the present invention is effervescent formulations which comprise the binder in the ratio of 1-8%, preferably in the ratio of 1-7%, most preferably in the ratio of 2-5% with respect to the total weight of the composition and cefdinir as the active agent.

The inventors have also found that when cefdinir: binder ratio is in the range of 5:1 to 1:3, preferably 4:1 to 2:1 in the effervescent formulations comprising cefdinir, the water solubility of cefdinir has increased.

Accordingly, another aspect of the present invention is the effervescent formulations comprising cefdinir wherein the ratio of cefdinir to the binder is in the range of 5:1 to 1:3, preferably in the range of 4:1 to 2:1.

The lubricant that can be used in effervescent formulations of the present invention can be selected from, but not limited to, a group comprising calcium stearate, magnesium stearate, polyethylene glycol, PEG 6000, polyvinyl alcohol, potassium benzoate, sodium benzoate. Preferably, PEG 6000 is used as lubricant in the formulations pertaining to the present invention.

The disintegrant that can be used in effervescent formulations of the present invention can be selected from, but not limited to, a group comprising carboxymethyl cellulose calcium, carboxymethyl cellulose sodium, microcrystalline cellulose, silicon dioxide, croscarmellose sodium, crospovidone, hydroxypropyl cellulose, methyl cellulose, povidone, magnesium aluminum silicate and starch or combinations thereof.

The diluent that can be used in effervescent formulations of the present invention can be selected from, but not limited to, a group comprising calcium carbonate, calcium sulphate, dibasic calcium phosphate, tribasic calcium phosphate, calcium phosphate, calcium sulphate, microcrystalline cellulose, lactose, magnesium carbonate, magnesium oxide, maltodextrin, maltose, mannitol, sodium chloride, sorbitol, starch and xylitol or combinations thereof.

The effervescent acid that is used in effervescent formulations pertaining to the present invention can be

selected from organic acids such as citric acid, tartaric acid, malic acid, fumaric acid. Preferably, citric acid is used as effervescent acid.

The effervescent base that is used in effervescent formulations pertaining to the present invention can be selected from sodium hydrogen carbonate, sodium carbonate, potassium carbonate and potassium hydrogen carbonate. Preferably, sodium hydrogen carbonate is used as effervescent base.

In effervescent formulation of the present invention, cefdinir or its pharmaceutically acceptable salts, hydrates, solvates or combinations thereof can be used.

The effervescent formulation of the present invention can comprise 5-30% cefdinir or pharmaceutically acceptable solvates, hydrates, enantiomers, racemates, organic salts, inorganic salts, polymorphs, crystal and amorphous forms of cefdinir and 1-20% organic base; 1-8% binder; 0.1-2% lubricant; 0.1-8% taste regulating agents, 0-25% diluent; 0-15% disintegrant; 0-10% humectant; 0.2-6% coloring agent and/or flavoring agent, 10-60% effervescent acid and 15-50% effervescent base with respect to the total amount of unit dose.

In another aspect, the present invention relates to the processes which can be used for the preparation of effervescent formulations comprising pharmaceutically acceptable excipients in addition to cefdinir as the active agent.

According to this, the process in scope of the present invention comprises the granulation of the active agent cefdinir through conventional wet and/or dry granulation methods present in the prior art or mixing cefdinir and the other excipients through dry blending method and then pulverizing them and optionally compressing them in tablet compressing machine to obtain tablet forms.

According to another aspect of the present invention, the pharmaceutical formulation prepared according to the present invention is used in the manufacture of a medicament so as to be used in upper respiratory infections such as ear, nose, throat, otitis media, sinusitis, tonsillitis, pharyngitis; lower respiratory tract infections such as pyelonephritis, cystitis and urethritis; skin or soft tissue infections such as froncle, pyoderma, impetigo; in the treatment and prophylaxis of gonorrhea and lyme diseases.

**Example 1:** Formulation and process for the preparation of effervescent tablet

| | % of amount present in unit dose |
|---|---|
| Cefdinir | 13% |
| Organic Base | 6% |
| Effervescent Acid | 41% |
| Effervescent Base | 28% |
| Binder | 3% |
| *Sodium chloride | 4.5% |
| *Sucralose | 1.5% |
| Lubricant | 2% |
| Coloring Agent | 1% |
| Flavoring Agent | 2% |
| * Sodium chloride: sucralose ratio is 3:1. | |

The formulation to be used in scope of said invention comprises granulating sodium hydrogen carbonate and cefdinir with aqueous organic base solution; then adding citric acid and sodium chloride into the obtained granule and granulating the mixture obtained with binder solution; following this, adding sucralose, lubricant, coloring agent and flavoring agent to the granule mixture obtained and mixing it again; then compressing the obtained mixture in tablet compressing machine to obtain tablets.

**Comparative Example 1:** Formulation and process for the preparation of effervescent tablet

| | % of amount present in unit dose |
|---|---|
| Cefdinir | 13% |
| Organic Base | 6% |
| Effervescent Acid | 38% |
| Effervescent Base | 30% |
| Binder | 3.5% |
| *Sodium chloride | 4% |
| * Sucralose | 0.5% |
| Lubricant | 1.5% |
| Coloring Agent | 1.5% |
| Flavoring Agent | 2% |
| *Sodium chloride: sucralose ratio is 8:1. | |

The formulation comprises granulating sodium hydrogen carbonate and cefdinir with aqueous organic base solution; then adding citric acid and sodium chloride into the obtained granule and granulating the mixture obtained with binder solution; following this, adding sucralose, lubricant, coloring agent and flavoring agent to the granule mixture obtained and mixing it again; then compressing the obtained mixture in tablet compressing machine to obtain tablets.

**Comparative Example 2:** Formulation and process for the preparation of effervescent tablet

| | % of amount present in unit dose |
|---|---|
| Cefdinir | 15% |
| Organic Base | 6% |
| Effervescent Acid | 38% |
| Effervescent Base | 31% |
| Binder | 3.5% |
| *Sodium chloride | 2.5% |
| Lubricant | 1.5% |
| Coloring Agent | 0.5% |
| Flavoring Agent | 2% |
| *Only sodium chloride is used a s taste regulating agent | |

The formulation comprises granulating sodium hydrogen carbonate and cefdinir with aqueous organic base solution; then adding citric acid and sodium chloride into the obtained granule and granulating the mixture obtained with binder solution; following this, adding lubricant, coloring agent and flavoring agent to the granule mixture obtained and mixing it again; then compressing the obtained mixture in tablet compressing machine to obtain tablets.

**Comparative Example 3:** Formulation and process for the preparation of effervescent tablet

| | % of amount present in unit dose |
|---|---|
| Cefdinir | 12% |

(continued)

| | % of amount present in unit dose |
|---|---|
| Organic Base | 6% |
| Effervescent Acid | 41.5% |
| Effervescent Base | 32% |
| Binder | 3.0% |
| *Sucralose | 1.5% |
| Lubricant | 1.5% |
| Coloring Agent | 0.5% |
| Flavoring Agent | 2% |
| *Only sucralose is used as taste regulating agent | |

The formulation comprises granulating sodium hydrogen carbonate and cefdinir with aqueous organic base solution; then adding citric acid into the obtained granule and granulating the mixture obtained with binder solution; following this, adding sucralose, lubricant, coloring agent and flavoring agent to the granule mixture obtained and mixing it again; then compressing the obtained mixture in tablet compressing machine to obtain tablets.

**Results:**

The inventors have studied on the dissolution data and bitterness values of the compositions illustrated in each example given above. Based on the studies on the comparison of their dissolution data and the bitterness values, the results shown in Tables 1-6 are obtained. Table 1 indicates the comparative data of dissolution time of the compositions for each example. In Table 2 to Table 5, the results of the bitterness values of each cefdinir compositions which are calculated according to the European Pharmacopoeia 5.0 (2.8.15, pg. 221) are shown.

**Table 1:** Comparative Data of Dissolution of Effervescent Tablets Comprising Cefdinir

| Sample | Dissolution Time (') minute ('') second |
|---|---|
| Composition of Example 1 | 2'25'' |
| Composition of Comparative Example 1 | 4'25'' |
| Composition of Comparative Example 2 | 5'32'' |
| Composition of Comparative Example 3 | 6'11'' |

The inventors have observed that the optimum dissolution time is less than 5 minutes for the effervescent formulations comprising cefdinir.

The results of the dissolution data of compositions of Example 1, Comparative example 1, Comparative example 2, Comparative example 3 clearly indicate that the effervescent tablet of the present invention in which sodium chloride and sucralose are used as two different taste regulating agent and the ratio of sodium chloride to sucralose is in the range of 5:1 to 1:1, has a dissolution time that is less than 5 minutes. This shows that the effervescent tablets prepared according to the present invention is more preferable than the other tablets via their higher dissolution rate.

**Table 2:** Results of the Taste Trial for Cefdinir Composition of Example 1

| Panelist* | k* | Y* | X* | Bitterness Value* |
|---|---|---|---|---|
| 1 | 0.88 | 100 | 8 | 110 |
| 2 | 0.72 | 100 | 6 | 120 |
| 3 | 0.8 | 10000 | 3 | 26667 |
| 4 | 0.84 | 100 | 3 | 280 |
| 5 | 0.72 | 10000 | 8 | 9000 |

(continued)

| Panelist* | k* | Y* | X* | Bitterness Value* |
|---|---|---|---|---|
| 6 | 0.76 | 50000 | 3 | 126667 |
| Average | | | | **27141** |
| Panelist*: a member of a taste panel of six people<br>k*: the correction factor for each panelist<br>Y*: Dilution Factor (DF) of solution D which is a diluted solution having still a bitter test<br>X*: number of milliliters of solution D which, when diluted to 10 ml with water, still has a bitter taste | | | | |

Bitterness value: a value calculated by using the expression of $\frac{Y \times k}{X \times 0.1}$

**Table 3:** Results of the Taste Trial for Cefdinir Composition of Comparative Example 1

| Panelist* | k* | Y* | X* | Bitterness Value* |
|---|---|---|---|---|
| 1 | 0.88 | 100 | 3 | 267 |
| 2 | 0.72 | 10000 | 6 | 12000 |
| 3 | 0.8 | 10000 | 6 | 13333 |
| 4 | 0.84 | 50000 | 2 | 210000 |
| 5 | 0.72 | 50000 | 3 | 120000 |
| 6 | 0.76 | 10000 | 2 | 38000 |
| Average | | | | **43600** |

**Table 4:** Results of the Taste Trial for Cefdinir Composition of Comparative Example 2

| Panelist* | k* | Y* | X* | Bitterness Value* |
|---|---|---|---|---|
| 1 | 0.88 | 50000 | 3 | 146667 |
| 2 | 0.72 | 10000 | 3 | 24000 |
| 3 | 0.8 | 50000 | 3 | 133334 |
| 4 | 0.84 | 10000 | 3 | 28000 |
| 5 | 0.72 | 10000 | 2 | 36000 |
| 6 | 0.76 | 10000 | 1.5 | 50667 |
| Average | | | | **69778** |

**Table 5:** Results of the Taste Trial for Cefdinir Composition of Comparative Example 3

| Panelist* | k* | Y* | X* | Bitterness Value* |
|---|---|---|---|---|
| 1 | 0.88 | 10000 | 2 | 44000 |
| 2 | 0.72 | 10000 | 3 | 24000 |
| 3 | 0.8 | 50000 | 3 | 133334 |
| 4 | 0.84 | 10000 | 2 | 42000 |
| 5 | 0.72 | 100000 | 3 | 240000 |
| 6 | 0.76 | 10000 | 1.5 | 50667 |
| Average | | | | **89000** |

Taste trials were performed by a taste panel comprising 6 persons. The average bitterness value of each effervescent composition of Example 1, Comparative example 1, Comparative example 2, Comparative example 3 are calculated according to the European Pharmacopoeia 5.0. Six panelists tasted the solutions of compositions prepared according to the method described in European Pharmacopoeia 5.0. The results are represented in Tables 2-5 and they clearly indicate that average bitterness value of the composition of example 1 is the lowest one. Therefore, it can be deduced that effervescent tablets formulated according to the present invention in which sodium chloride and sucralose are used as two different taste regulating agent and the ratio of sodium chloride to sucralose is in the range of 5:1 to 1:1 shown in example 1 is much preferred via its more pleasant taste.

## Claims

1. A pharmaceutical formulation formulated in effervescent form comprising cefdinir as the active agent **characterized in that** the combination of sodium chloride and sucralose is used as taste regulating agent and the ratio of sodium chloride to sucralose is in the range of 5:1 and 1:1.

2. The pharmaceutical formulation according to claim 1, wherein cefdinir is present in the form of its solvates, hydrates, enantiomers, racemates, organic salts, inorganic salts, polymorphs, in crystal and amorphous forms or in free form and/or combination thereof.

3. The pharmaceutical formulation according to claims 1-2, wherein the ratio of cefdinir to the combination of sodium chloride and sucralose is in the range of 10:1 to 1:1.

4. The pharmaceutical formulation according to claims 1-3, wherein organic base is used in addition to cefdinir and the taste regulating agent and the ratio of cefdinir to the organic base is in the range of 5:1 to 1:5.

5. The pharmaceutical formulation according to claim 4, wherein primary amines, secondary amines, tertiary amines and/or heterocyclic compounds containing nitrogen can be used as the organic base.

6. The pharmaceutical formulation according to claim 5, wherein organic base that is to be used in the formulation is selected from a group comprising ethanolamine, isopropanolamine, 1-dioxy-1-methylamino-sorbitol, 1-dioxy-1-methylamino-D-glucitol, tris(hydroxymethyl)aminomethane, N-(Tri(hydroxymethyl)methyl)glycine, N,N-Bis(2-hydroxyethyl)glycine, 2-methyl aminophenol.

7. The pharmaceutical formulation according to claims 1 - 6, wherein pharmaceutically acceptable excipients selected from a group comprising binders, lubricants, humectants, disintegrants, diluents, and effervescent acid - base couple in addition to cefdinir, combination of sodium chloride and sucralose and organic base.

8. The pharmaceutical formulation according to claim 7, wherein said formulation comprises 1-8% binder.

9. The pharmaceutical formulation according to claims 1-8, wherein cefdinir: binder ratio is in the range of 5:1 to 1:3.

10. The pharmaceutical formulation according to claim 7, wherein the effervescent acid is selected from organic acids such as citric acid, tartaric acid, malic acid, fumaric acid.

11. The pharmaceutical formulation according to claim 7, wherein the effervescent base is selected from basic agents such as sodium hydrogen carbonate, sodium carbonate, potassium carbonate and potassium hydrogen carbonate.

12. The pharmaceutical formulation according to claims 1-11, wherein said formulation comprises 5-30% cefdinir or pharmaceutically acceptable solvates, hydrates, enantiomers, racemates, organic salts, inorganic salts, polymorphs, crystal and amorphous forms of cefdinir and 1-20% organic base; 1-8% binder; 0.1-2% lubricant; 0.1-8% taste regulating agent, 0-25% diluent; 0-15% disintegrant; 0-10% humectant; 0.2-6% coloring agent and/or flavoring agent, 10-60% effervescent acid and 15-50% effervescent base with respect to the total amount of unit dose with respect to the total amount of unit dose.

13. The process for preparation of the pharmaceutical formulation according to the preceeding claims, wherein said process comprises mixing the components through dry blending method and powderizing them or granulation of the active agent cefdinir and various excipients through conventional wet and/or dry granulation methods and optionally compressing these in tablet compressing machine to obtain tablet forms.

## Patentansprüche

1. Die in Brauseform formulierte pharmazeutische Formulierung, die Cefdinir als Wirkstoff umfasst und **dadurch gekennzeichnet, dass** die Kombination aus Natriumchlorid und Sucralose als Geschmacksregulierungsmittel verwendet wird und das Verhältnis von Natriumchlorid zu Sucralose im Bereich von 5:1 und 1:1 liegt.

2. Die pharmazeutische Formulierung nach Anspruch 1, wobei Cefdinir in Form seiner Solvate, Hydrate, Enantiomere, Racemate, organischen Salze, anorganischen Salze, Polymorphen, in Kristall und amorphen Formen oder in freier Form und/oder einer Kombination davon vorhanden ist.

3. Die pharmazeutische Formulierung nach den Ansprüchen 1-2, wobei das Verhältnis von Cefdinir zu der Kombination von Natriumchlorid und Sucralose im Bereich von 10: 1 bis 1: 1 liegt.

4. Die pharmazeutische Formulierung nach den Ansprüchen 1-3, wobei organische Base zusätzlich zum Cefdinir und dem Geschmack-Regulierungsmittel verwendet wird und das Verhältnis von Cefdinir zur organischen Base im Bereich von 5: 1 bis 1: 5 liegt.

5. Die pharmazeutische Formulierung nach Anspruch 4, wobei primäre Amine, sekundäre Amine, tertiäre Amine und/oder heterocyclische Verbindungen, die Stickstoff enthalten, als organische Base verwendet werden können.

6. Die pharmazeutische Formulierung nach Anspruch 5, wobei organische Base, die in der Formulierung verwendet wird, aus einer Gruppe bestehend aus Ethanolamin, Isopropanolamin, 1-deoxy-1-methylamino-sorbitol,1-deoxy-1-methylamino-D-glucitol,tris(hydroxymethyl)aminomethane,N (Tri(hydroxymethyl)methyl)glycine,N,N-Bis(2-hydroxyethyl) glycin, 2-Methyl-aminophenol ausgewählt wird.

7. Die pharmazeutische Formulierung nach den Ansprüchen 1-6, wobei pharmazeutisch annehmbare Hilfsstoffe aus einer Gruppe bestehend aus Bindemitteln, Gleitmitteln, Feuchthaltemittel, Sprengmittel, Verdünnungsmittel und Brause- Säuren-Basen-Paar neben Cefdinir, Kombination aus Natriumchlorid und Sucralose und organischer Base ausgewählt werden.

8. Die pharmazeutische Formulierung nach Anspruch 7, wobei die erwähnte Formulierung 1-8% Bindemittel beinhaltet.

9. Die pharmazeutische Formulierung nach den Ansprüchen 1-8, wobei Cefdinir: Bindemittel-Verhältnis im Bereich von 5: 1 bis 1: 3 liegt.

10. Die pharmazeutische Formulierung nach Anspruch 7, wobei die Brausesäure aus organischen Säuren wie Zitronensäure, Weinsäure , Äpfelsäure , Fumarsäure ausgewählt wird.

11. Die pharmazeutische Formulierung nach Anspruch 7, wobei die Brausebasis aus basischen Mitteln wie Natriumhydrogencarbonat, Natriumcarbonat, Kaliumcarbonat und Kaliumhydrogencarbonatausgewähltwird.

12. Die pharmazeutische Formulierung nach den Ansprüchen 1-11, wobei die erwähnte Formulierung 5-30% von Cefdinir oder pharmazeutisch annehmbaren Solvaten, Hydraten, Enantiomere, Racemate, organischen Salzen, anorganischen Salzen, Polymorphen, Kristall- und amorphen Formen von Cefdinir und 1-20% von organischer Basis; 1-8% von Bindemittel; 0,1-2% von Schmiermittel; 0,1-8% von Geschmack- Regulierungsmittel, 0-25% von Verdünnungsmittel; 0-15% von Sprengmittel; 0-10% von Feuchthaltemittel; 0,2-6% von Färbemittel und/oder Aromastoff, 10-60% von Brausesäure und 15-50% von Brausebasis in Bezug auf die Gesamtmenge der Dosierungseinheit bezüglich der Gesamtmenge der Dosierungseinheit umfasst.

13. Verfahren zur Herstellung der pharmazeutischen Formulierung nach vorhergehenden Ansprüchen, wobei das erwähnte Verfahren das Mischen der Komponenten durch Trockenmischverfahren und Pulverisieren oder Granulation des Wirkstoffes, Cefdinir und derverschiedenen Hilfsstoffe durch herkömmliche nasse und/oder trockene Granulationsmethoden und gegebenenfalls Komprimieren diese in Tablettenpressmaschineumfasst, um Tablettenformen zu erhalten.

## Revendications

**1.** Une formulation pharmaceutique formulée dans une forme effervescente comprenant cefdinir comme l'agent actif, **caractérisé en ce que** la combinaison du chlorure de sodium et le sucralose est utilisé comme l'agent régulateur de goût et le rapport du chlorure de sodium au sucralose est dans la plage de 5:1 et 1:1.

**2.** Une formulation pharmaceutique selon la revendication 1, dans laquelle, cefdinir est présent sous la forme de son solvates, hydrates, énantiomères, racémates, sels organiques, sels inorganiques, polymorphes, sous la forme cristalline et amorphe ou bien sous la forme libre et/ou d'une combinaison de ceux-ci.

**3.** La formulation pharmaceutique selon les revendications 1-2, dans laquelle le rapport du cefdinir à la combinaison du chlorure de sodium et le sucralose est dans la plage de 10:1 à 1:1.

**4.** La formulation pharmaceutique selon les revendications 1-3, dans laquelle la base organique est utilisée en plus du cefdinir et de l'agent régulateur de goût et le rapport du cefdinir à la base organique est dans la plage de 5:1 à 1:5.

**5.** La formulation pharmaceutique selon la revendication 4, dans laquelle les amines primaires, les amines secondaires, les amines tertiaires et/ou des composés hétérocycliques contenant de l'azote peuvent être utilisés comme la base organique.

**6.** La formulation pharmaceutique selon la revendication 5, dans laquelle la base organique à utiliser dans la formulation est choisi d'un group comprenant l'éthanolamine, l'isopropanolamine, le 1-dioxy-1-méthylamino sorbitol, 1-dioxy-1-méthylamino-D-glucitol, le tris (hydroxyméthyl) aminométhane, N- (tri (hydroxyméthyl) méthyl) glycine, la N, N-bis (2-hydroxyéthyl) glycine, le 2-méthyl aminophenol.

**7.** La formulation pharmaceutique selon les revendications 1-6, dans laquelle les excipients pharmaceutiquement acceptables sont choisi d'un group comprenant des liants, des lubrifiants, des humectants, des désintégrants, des diluants, et un couple d'acide-base effervescent en plus de cefdinir, la combinaison du chlorure de sodium et le sucralose et la base organique.

**8.** La formulation pharmaceutique selon la revendication 7, dans laquelle ladite formulation comprend le liant dans un rapport de 1-8%.

**9.** La formulation pharmaceutique selon les revendications 1-8, dans laquelle le rapport du cefdinir au liant est dans la plage de 5:1 à 1:3.

**10.** La formulation pharmaceutique selon la revendication 7, dans laquelle l'acide effervescent est choisi parmi les acides organiques tels qu'acide citrique, acide tartrique, acide malique, acide fumarique.

**11.** La formulation pharmaceutique selon la revendication 7, dans laquelle la base effervescente est choisie parmi les agents basiques tels que le carbonate d'hydrogène de sodium, le carbonate de sodium, le carbonate de potassium et le carbonate d'hydrogène de potassium.

**12.** La formulation pharmaceutique selon les revendications 1-11, dans laquelle ladite formulation comprend cefdinir dans un rapport de 5 à 30% ou des solvates pharmaceutiquement acceptables, des hydrates, des énantiomères, des racémates, des sels organiques, des sels inorganiques, des polymorphes, les formes cristalline et amorphe du cefdinir et de la base organique dans un rapport de 1-20%; liant dans un rapport de 1-8%; lubrifiant dans un rapport de 0.1-2% ; agent régulateur de goût dans un rapport de 0.1-8%, diluant dans un rapport de 0.25%; désintégrant dans un rapport de 0-15%; humectant dans un rapport de 0-10%; agent colorant et/ou agent aromatisant dans un rapport de 0.2-6%, acide effervescente dans un rapport de 10-60% et base effervescente dans un rapport de 15-50% par rapport à la quantité totale de la dose unitaire.

**13.** Un procédé pour préparation de la formulation pharmaceutique selon les revendications précédentes, dans lequel ledit procédé comprend mélanger des composants par mélanger à sec et les poudrer ou granuler l'agent actif, cefdinir, et les divers excipients par les méthodes conventionnels de granulation humide et/ou à sec et éventuellement comprimer ceux-ci dans une machine de compression afin d'obtenir les formes de comprimés.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- BE 897864